(19)

**Europäisches Patentamt**

**European Patent Office**

**Office européen des brevets**

(11) **EP 4 717 308 A1**

(12) **EUROPEAN PATENT APPLICATION**

(43) Date of publication:
01.04.2026 Bulletin 2026/14

(21) Application number: **25204593.5**

(22) Date of filing: **25.09.2025**

(51) International Patent Classification (IPC):
**A61N 5/10** (2006.01)

(52) Cooperative Patent Classification (CPC):
**A61N 5/103; A61N 5/1047;** A61N 5/1036

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **26.09.2024 US 202418897826**

(71) Applicant: **Siemens Healthineers International AG**
**6312 Steinhausen (CH)**

(72) Inventors:
• **VALENZUELA, Daniel**
**0440 Helsinki (FI)**
• **NURMINEN, Jussi**
**00410 Helsinki (FI)**
• **TALLINEN, Tuomas**
**02600 Espoo (FI)**
• **SABEL, Martin**
**6332 Hagendorn (CH)**
• **PELTOLA, Jarkko**
**04300 Tuusula (FI)**

(74) Representative: **Mathisen & Macara LLP**
**Charta House**
**30-38 Church Street**
**Staines-upon-Thames TW18 4EP (GB)**

(54) **RADIATION TREATMENT PLAN APPARATUS AND METHOD**

(57) When optimizing (202) a radiation treatment plan (113) for a particular patient (104) using a particular radiation treatment platform (114), which radiation treatment plan (113) has a plurality of treatment fields where a source of radiation (115) is moving according to a treatment arc, and wherein in at least one of the treatment fields the source of radiation (116) stops at at least one stopping point to deliver additional dose, a control circuit (101) can, as part of optimizing the radiation treatment plan (113), optimize (202) collimator rotation with respect to the at least one stopping point.

200

FOR A PARTICULAR PATIENT USING A PARTICULAR RADIATION TREATMENT PLATFORM, WHICH RADIATION TREATMENT PLAN HAS A PLURALITY OF TREATMENT FIELDS WHERE A SOURCE OF RADIATION IS MOVING ACCORDING TO A TREATMENT ARC, WHEREIN IN AT LEAST ONE OF THE TREATMENT FIELDS THE SOURCE OF RADIATION STOPS AT AT LEAST ONE STOPPING POINT TO DELIVER ADDITIONAL DOSE — 201

BY A CONTROL CIRCUIT

OPTIMIZING A RADIATION TREATMENT PLAN WHEREIN OPTIMIZING THE RADIATION TREATMENT PLAN INCLUDES OPTIMIZING COLLIMATOR ROTATION WITH RESPECT TO THE AT LEAST ONE STOPPING POINT — 202

OUTPUTTING AN OPTIMIZED RADIATION TREATMENT PLAN — 203

ADMINISTERING THERAPEUTIC RADIATION TO THE PARTICULAR PATIENT USING THE OPTIMIZED RADIATION TREATMENT PLAN — 204

FIG. 2

300

OPTIMIZING COLLIMATOR ROTATION WITH RESPECT TO THE AT LEAST ONE STOPPING POINT

ACCESSING A TWO-DIMENSIONAL MATRIX COMPRISING A COST MAP HAVING A FIRST AXIS THAT CORRESPONDS TO GANTRY ANGLES AND A SECOND AXIS THAT CORRESPONDS TO COLLIMATOR ANGLES, SUCH THAT CELLS IN THE TWO-DIMENSIONAL MATRIX STORE A COST THAT CORRESPONDS TO A GIVEN COMBINATION OF GANTRY ANGLE AND COLLIMATOR ANGLE, SUCH THAT COLLIMATOR ANGLE CONSTRAINTS FOR STATIC GANTRY ANGLES AND ARC START AND STOP ANGLES ARE MODELED AS FORBIDDEN OR HIGH-COST CELLS — 301

DETERMINING AN OPTIMAL PATH THROUGH THE TWO-DIMENSIONAL MATRIX FOR AT LEAST MOST APPLICABLE GANTRY ANGLES — 302

FIG. 3

EP 4 717 308 A1

## Description

TECHNICAL FIELD

These teachings relate generally to treating a patient's planning target volume with energy pursuant to an energy-based treatment plan and more particularly to optimizing an energy-based treatment plan.

BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Collimators, including multi-leaf collimators, are often employed to shape or to otherwise modulate the radiation beam. Multi-leaf collimators are comprised of a plurality of individual parts (known as "leaves") that are formed of a high atomic numbered material (such as tungsten) that can move independently in and out of the path of the radiation-therapy beam in order to selectively block (and hence shape) the beam. Typically the leaves of a multi-leaf collimator are organized in pairs that are aligned collinearly with respect to one another and which can selectively move towards and away from one another. A typical multi-leaf collimator has many such pairs of leaves, often upwards of twenty, fifty, or even one hundred such pairs.

In some application settings, one or more collimators can be selectively rotated. The rotation of a radiation beam collimator during radiation treatment can allow, for example, for more precise shaping of the radiation beam to match the contour of a targeted tumor, thereby optimizing dose delivery to the cancerous tissue while better sparing surrounding healthy tissue. By rotating the collimator, clinicians can also create non-coplanar beam arrangements that can further minimize exposure to sensitive structures and increase the treatment's conformality. Additionally, collimator rotation can be used in conjunction with other techniques like intensity-modulated radiation therapy (IMRT) to modulate the intensity of the radiation beam across the treatment field for even more tailored dose distributions.

SUMMARY OF INVENTION

In accordance with a first aspect of the invention, there is provided a method as defined by claim 1.

In accordance with a second aspect of the invention, there is provided an apparatus as defined by claim 9.

Optional features are defined by the dependent claims.

BRIEF DESCRIPTION OF DRAWINGS

The above needs are at least partially met through provision of the radiation treatment plan apparatus and method described in the following detailed description, particularly when studied in conjunction with the drawings, wherein:

> FIG. 1 comprises a block diagram as configured in accordance with various embodiments of these teachings;
> FIG. 2 comprises a flow diagram as configured in accordance with various embodiments of these teachings; and
> FIG. 3 comprises a flow diagram as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

DETAILED DESCRIPTION

Generally speaking, these various embodiments can serve to facilitate optimizing a radiation treatment plan for a particular patient using a particular radiation treatment platform, which radiation treatment plan has a plurality of treatment fields where a source of radiation is moving according to a treatment arc, and wherein in at least one of the treatment fields the source of radiation stops at at least one stopping point to deliver dose, such as additional dose.

In such an application setting, a control circuit can, as part of optimizing the radiation treatment plan, optimize collimator rotation with respect to the at least one stopping point.

By one approach, the rotatable collimator comprises a multi-leaf collimator, in which case, optimizing collimator rotation with respect to the at least one stopping point can comprise maximizing or determining optimal leaf positioning for the multi-leaf collimator.

By one approach, optimizing the collimator rotations can be undertaken (and completed, if desired) before initiating dose optimization iterations.

By one approach, optimizing the collimator rotation with respect to the at least one stopping point can comprise optimizing collimator rotation without physical constraints. In such a case, if desired, optimizing collimator rotation without physical constraints can further comprise optimizing by applying weighting that emphasizes the collimator angle at the at least one stopping point. By another approach, optimizing collimator rotation with respect to the at least one stopping point can comprise optimizing collimator rotation subject to physical constraints regarding any combination of one or more of: a collimator rotation value at a static angle, a collimator rotation value at an arc stop position, start/end arc positions of a beam-off sector, and/or a collimator rotation value at an arc start position.

By one approach, optimizing collimator rotation with respect to the at least one stopping point can comprise optimizing collimator rotation such that a patient target volume width in a direction coincident with collimator leaves is minimized; and in an optional example, such that a patient target volume width in a direction coincident with a direction of movement of collimator leaves is minimized. By another approach, in lieu of the foregoing or in combination therewith, optimizing collimator rotation with respect to the at least one stopping point can comprise optimizing the collimator rotation to favor orienting collimator leaves to yield an emphasized effective radiation modulation of a target volume.

By one approach, these teachings will accommodate optimizing collimator rotation with respect to the at least one stopping point by accessing a two-dimensional matrix comprising a cost map having a first axis that corresponds to gantry angles and a second axis that corresponds to collimator angles, such that cells in the two-dimensional matrix store a cost that corresponds to a given combination of gantry angle and collimator angle, such that collimator angle constraints for static gantry angles and arc start and stop angles are modeled as forbidden or high-cost cells. So configured, these teachings will accommodate determining an optimal path through the two-dimensional matrix for at least most applicable gantry angles.

These teachings will then accommodate outputting a resultant optimized radiation treatment plan and administering therapeutic radiation to the particular patient using the optimized radiation treatment plan.

Many prior art approaches disregard at least some requirements of a radiation treatment plan where the gantry stops (at least once and possibly more times) to deliver additional dose during a given radiation treatment session. The present teachings can handle multiple fields simultaneously so that the resulting collimator rotation trajectories can be different. This contrasts sharply with some prior art approaches that can often result in scenarios where two or more arcs have identical collimator rotations, a result that can run contrary to established clinical practice.

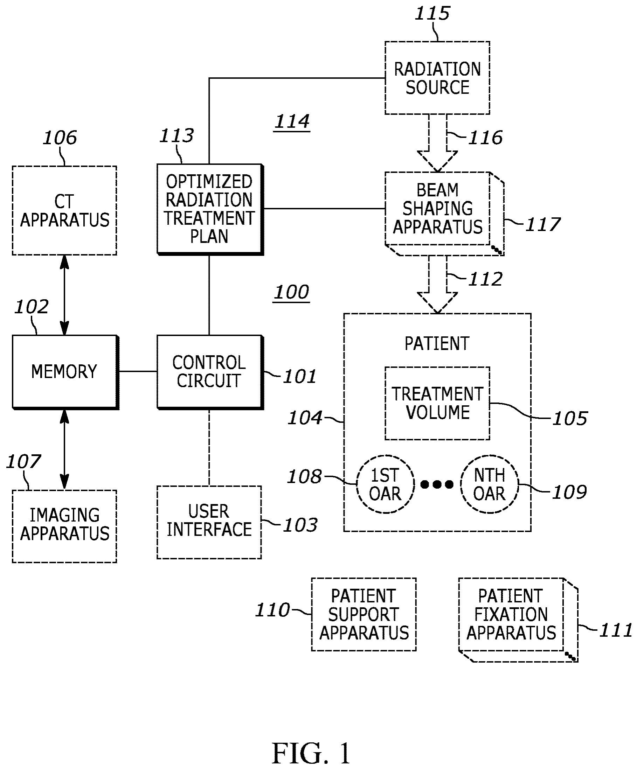

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway. In addition, these teachings will accommodate a radiation treatment platform where the source of radiation can stop at at least one stopping point during a given radiation treatment session to deliver additional dose per the dictates of a corresponding radiation treatment plan.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses. These beam-shaping apparatuses 117 may comprise such things as jaws, multi-leaf collimators, and so forth and serve to provide selective energy shaping and/or energy modulation as desired. One or more such beam-shaping apparatuses 117 may be selectively rotatable during the radiation treatment session.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

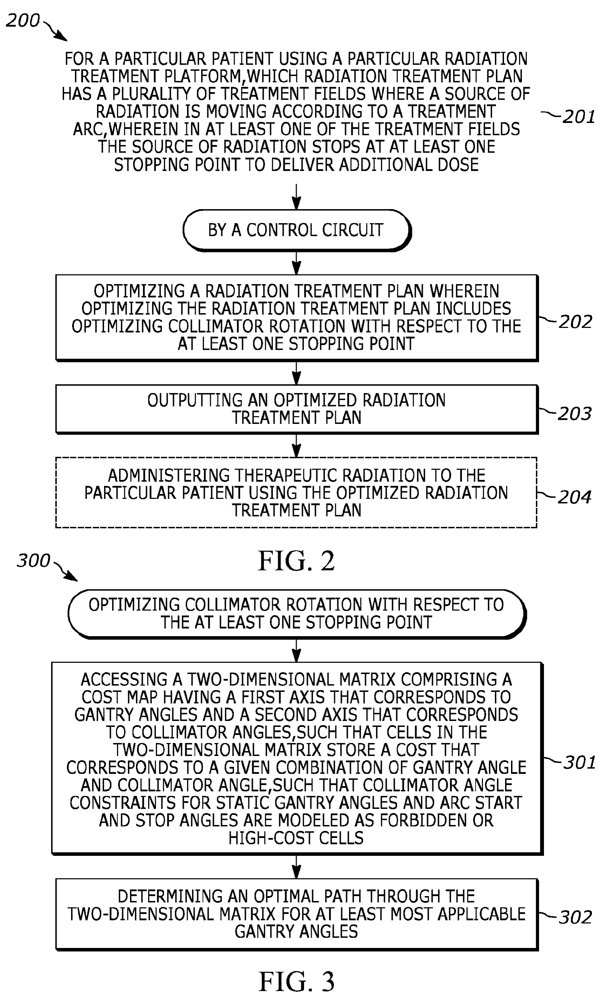

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

As denoted by reference numeral 201, this process 200 in this illustrative example presumes optimizing a radiation treatment plan for a particular patient using a particular radiation treatment platform, which radiation treatment plan has a plurality of treatment fields where a source of radiation is moving according to a treatment arc, wherein in at least one of the treatment fields the source of radiation stops at at least one stopping point to deliver additional dose.

At block 202, the control circuit optimizes a radiation treatment plan. In addition to other functionality that typifies such optimization, pursuant to this process 200, optimizing the radiation treatment plan further includes optimizing collimator rotation with respect to the aforementioned at least one stopping point. By way of a simple illustrative example, if the source of radiation stops at three separate stopping points to deliver dose or additional dose, this process 200 can provide for optimizing collimator rotation with respect to each of those stopping points.

For the sake of an illustrative example, and without intending to suggest any limitations in these regards, this description will presume that the rotatable collimator comprises a rotatable multi-leaf collimator. Accordingly, optimizing collimator rotation with respect to the at least one stopping point will comprise maximizing or determining optimal leaf positioning for the multi-leaf collimator. By one approach, optimizing collimator rotation with respect to the at least one stopping point can comprise optimizing collimator rotation such that a patient's target volume within a direction coincident with leaves of the collimator are minimized, or, such that a patient's target volume within a direction coincident with a direction of movement of leaves of the collimator are minimized. Generally speaking, these teachings can serve to optimize collimator rotation to favor orienting collimator leaves to yield an emphasized effective radiation modulation of a target volume.

Optimization can include a variety of steps and activities. One common activity is to optimize dosing via an iterative optimization process. By one approach, these teachings will accommodate optimizing the radiation treatment plan by optimizing collimator rotations before initiating any such dose optimization iterations.

By one approach optimizing collimator rotation can be conducted without any physical constraints being imposed. In such a case, these teachings can accommodate optimizing collimator rotation by applying weighting that emphasizes the collimator angle at the at least one stopping point.

By another approach, optimizing collimator rotation with respect to the at least one stopping point can instead comprise optimizing collimator rotation subject to physical constraints. These teachings will accommodate accounting for any of a variety of physical constraints. Examples include, but are not limited to, any combination of one or more of: a collimator rotation value at a static angle, a collimator rotation value at an arc stop position, start/end arc positions of a beam-off sector, and/or a collimator rotation value at an arc start position.

Referring momentarily to FIG. 3, this figure presents an illustrative process 300 for optimizing collimator rotation with respect to the at least one stopping point.

At block 301, the control circuit 101 accesses a two-dimensional matrix that comprises a cost map having a first axis that corresponds to gantry angles and a second axis that corresponds to collimator angles. So configured, cells in the two-dimensional matrix store a cost that corresponds to a given combination of gantry angle and collimator. Accordingly, collimator angle constraints for static gantry angles, arc start angles, and arc stop angles can be modeled as forbidden or high-cost cells.

At block 302, the control circuit 101 can then determine an optimal path through the two-dimensional matrix. Since there may not be one path that is literally optimal for every applicable gantry angle individually, by one approach these teachings will accommodate determining a path through the two-dimensional matrix that is optimal in the sense that the sum of the costs of the individual angles is optimal.

Referring again to FIG. 2, at block 203, the control circuit 101 outputs an optimized radiation treatment plan. At optional block 204, therapeutic radiation can be administered to the aforementioned particular patient using the aforementioned particular radiation treatment platform and using the optimized radiation treatment plan yielded by the foregoing steps.

Further details that comport with these teachings will now be presented. It will be understood that the specific details of these examples are again intended to serve an illustrative purpose and are not intended to suggest any particular limitations with respect to these teachings.

For the sake of an illustrative example, this description may be seen as (but does not have to be seen as) presuming that the trajectory defining how the gantry rotates is part of the input, given either by the user or by another independent algorithm. The same holds for other aspects of the input geometry, such as the patient support surface and isocenter. The input can be presented as a set of control point sequences. Control points refer to specific positions or angles at which a radiation beam is turned on or off during treatment. Control points typically serve to help shape the radiation beam and to optimize the dose distribution to target the tumor while sparing surrounding healthy tissues.

In this example, the output is a set of control point sequences, in which the collimator values have been optimized, and all other values remain unaffected. The resulting optimized collimator rotation will lead to a dynamic collimator rotation for the parts of the field where the gantry is rotating and a static optimized collimator angle for the control points where the gantry rotation is pausing.

This description also assumes the existence of a separate plan optimization algorithm that will optimize the leaf positions to provide the best possible plan dose based on the user requirements (referred to herein as "the leaf optimizer"). The underlying idea of the immediate approach described here is to identify collimator angles that allow the leaf optimizer to obtain better results. In an optional example, collimator angles are optimized in order to improve leaf optimizer results.

Optimizing the collimator for a single field

Cost functions to optimize collimator rotation

The collimator rotation can be optimized by minimizing a cost function that depends on the control point geometry and patient anatomy. In the following description, the XY coordinate system describes the beam's eye view (BEV), so that the X axis is aligned with the direction of the leaf movement and the Y axis is orthogonal to the X axis, correlated with the number of leaves. The target is projected onto the BEV, and the projection is cropped considering the maximum possible aperture of the corresponding jaw(s). The magnitude of the projection is described by a discretized volume function $V(x, y)$. Thus, $V(x, y)$ describes the total target volume that gets projected into the point $(x,y)$ in the BEV. The effect of collimator rotation may be considered by including a rotation parameter $\alpha$ in the volume function, which becomes $V(\alpha, x, y)$; the effect of $\alpha$ is just to rotate the original projection $V(x,y)$ by $\alpha$ degrees in the BEV.

The following alternative cost functions can be employed, alone or in any combination, as desired.

Maximizing the number of leaves that can reach and modulate the dose in the target. Optionally, this involves maximizing the number of leaves whose position can be changed so that dose can reach, and be modulated for, the target. If one assumes leaf thickness to be 1, this is equivalent to the extent of the target projection onto the Y axis.

Considering not only the extent but also the magnitude of target projection onto the Y axis, this cost can aim to maximize the number of leaves that can modulate the target, but it also gives more emphasis to thicker parts of the target.

Minimizing the width of the target in the X direction. Minimizing the target width in the direction of leaf motion tends to minimize the amount of required leaf travel. Also, the target projection in the orthogonal Y direction tends to be maximized, again increasing the number of leaves available for modulation.

Minimizing the volume-weighted squared width of the target in the X direction. Taking the volume into account in this way can emphasize the regions where the target projection is larger.

By one approach, the cost function can be defined as

$$C(\alpha) = \sum_k c(y_k).$$

where

$$c(y_k) = \begin{cases} 1 & \text{if } \max_i V(\alpha, x_i, y_k) \geq 0 \\ 0 & \text{if } \max_i V(\alpha, x_i, y_k) = 0 \end{cases}$$

By another approach, the definition can be the same, except that

$$c(y_k) = \sqrt{\sum_i V(\alpha, x_i, y_k)^2}$$

By yet another approach, the cost is just the maximum width in the X direction, i.e.

$$C(\alpha) = \max_{m,n} (x_m - x_n)$$

where the X coordinates must satisfy

$$V(\alpha, x_n, y_j) \geq 0, \quad V(\alpha, x_m, y_k) \geq 0$$

for some choice of j and k.

And lastly, by yet another approach, one can define the geometric mean of the target in the X direction as

$$\mu = \frac{\sum_{i,j} x_i V(\alpha . x_i . y_j)}{\sum_{i,j} V(\alpha . x_i . y_j)}$$

The cost can then be computed as

$$C(\alpha) = \sum_{i,j} V(\alpha . x_i . y_j)(x_i - \mu)^2$$

Finding an optimal collimator rotation for the entire arc

Using the chosen metric, a (gantry, collimator) two-dimensional map can be built. For example, one dimension may represent the gantry angle while the other dimension may represent the collimator angle. For each gantry value in the input sequence and for each possible collimator angle value, the projection of the target volume into the BEV is computed. Then the cost corresponding to the projection is evaluated and stored in the relevant corresponding map cell.

One could simply choose the optimal collimator angle per each gantry angle in the input sequence. However, that approach would not consider the physical restrictions on the arc itself. One established trajectory-finding method is Dijkstra's algorithm, which can identify the shortest path in a graph. One can consider the gantry-collimator map, such as a {gantry angle}-{collimator angle} map, as a graph where each cell in the map corresponds to a node, with edges from each cell to its immediate neighbors on the right that are reachable with the constraints of maximum collimator rotation speed and the maximum gantry rotation speed. In this way, Dijkstra's algorithm will provide a collimator trajectory that will minimize the total cost while respecting the movement limits, for example while respecting the movement limits of the collimator and/or gantry.

Optimizing the collimator for dynamic fields

Case 1: static angles with user-input collimator angles

These teachings support the use case where the user defines a specific collimator angle for each static gantry position in a dynamic field, or more generally, the collimator angle of any subset of control points from the input. Optionally, the user may constrain a collimator angle for a gantry position; optimization may then proceed as above with this constraint.

For each user-defined gantry-collimator pair constraint, $(g_i, c_i)$, these teachings will accommodate blocking of all the collimator values associated with $g_i$, with the exception of $c_i$. Then this approach can operate as described above.

Optimizing the collimator for dynamic fields

Case 2: static angles without input constraints

By one approach, these teachings support the use case where the user does not define any specific collimator angle for each static gantry position. Instead, each static gantry position can be assumed to be more important than any other gantry angle of the corresponding arc. In that case, the static gantry angles can be treated as high priority angles, and to ensure near-optimality at these gantry positions, cells with relatively high cost at the static gantry position can be forbidden. The constraints can be set loose enough so that transitions between static gantry positions do not require unduly steep changes in collimator rotation. Forbidden collimator angles for a static gantry position can be defined, for example, as cells whose cost exceeds a specified fraction of mean cost of cells with that gantry angle. In an optional example, forbidden collimator angles are not available during optimization. By another approach, the cost of all cells at the static gantry angles may be scaled by a desired priority factor to prioritize static gantry angles without strictly forbidding any particular collimator angles.

Optimizing multiple fields together

When the radiation treatment plan has multiple fields, these teachings will accommodate working in two different modalities, depending on the input geometry. Differing workflows may be beneficially utilized, as explained below.

Equivalent-geometry fields

Two (or more) fields having identical input geometry (defined here as having the same isocenter, the same patient support surface angle, and the same field opening (typically defined by the jaws position)) can be treated as a special case. In such a setup, these teachings can be applied to aim to generate distinct collimator angles for the fields, as the other geometrical aspects are the same. In this way, the final plan optimization approach can have better modulation of the dose.

To achieve this, when optimizing the second (or further) collimator angle, one can apply penalties to the map cells that are close (that is, within some user-established or default tolerance) to the previously optimized collimator trajectories.

Collimator redundancy penalty

These teachings can support using different functions to define the amount of penalty to apply to each map cell. By one approach, for any cell $(g_i, c_i)$ in the map, let $(\hat{g}_i, \hat{c}_i)$ be the closest point in the previous trajectory (when there is a previous trajectory). Costs for the penalized map cells can be computed as follows:

$$C(g_i, c_i) = C(g_i, c_i) + P(c_i, \hat{c}_i)$$

Some examples of penalty functions can be represented as follows:

$$P_1\,(c_i, \hat{c}_i) = \; B * (1 - \frac{|c_i - \hat{c}_i|}{t}),$$

when $|c_i - \hat{c}_i| < t$
(and optionally, = 0, when $|c_i - \hat{c}_i| \geq t$)

$$P_2\,(c_i, \hat{c}_i) = \; B * \cos\,(c_i - \hat{c}_i)$$

where B is a constant indicating the base value of the penalty.

In both of the foregoing cases the penalty is maximum when $c_i = \hat{c}_i$, with the penalty decreasing as the difference between the collimator angles increases.

In the case of $P_1$, the penalty can be applied only within a threshold t, and it can decrease linearly from B to zero when the threshold is met, and it can evaluate to zero elsewhere.

In the case of $P_2$, the penalty can proceed according to the dot product between the collimator angles, and it can also evaluate to B when the values are identical, but it can decrease according to the *cosine* function, reaching zero when the angles are perpendicular. The rationale behind this is because when the collimator angles are perpendicular, the leaf optimizer will have the most degrees of freedom to modulate the plan. In both cases B is the base value of the penalty calculated based on the existing gantry-collimator map (e.g., the average of the cells), prior to the application of the penalties. In one optional example, the base value may comprise the average of the cells of the gantry-collimator map. Optionally, the penalty may take a value ranging from the base value to zero.

Higher number of trajectories

When the number of arc trajectories is high (say, four or more), and if one were to just apply the previously described process iteratively, the costs in the map could eventually be dominated by the penalties. As one potential underlying preference may be to keep the original map as the dominant cost and to use the penalties as a secondary criterion, these teachings will accommodate using a budget-based approach for the penalties of all previously-computed trajectories. By one approach, then, when optimizing the collimator for the k-th arc, instead of starting from the map of the (k-1)th arc, these teachings will accommodate starting from the original map and applying a penalty for the k-1 previous trajectories, where each of them will be weighted by a factor a=1/(k-1).

Negative Penalties

If there are certain trajectories that are to be preferred, and the process encourages the trajectories to stay close (instead of being distinct) to a previously existing trajectory, these teachings will accommodate adding a negative penalty to the cells in the maps that are close to it. The effect in this case would be that subsequent trajectories will be more likely to follow the same trajectories as those who received a negative penalty. In an optional example, a negative penalty may be assigned to cells in the gantry-collimator map to reinforce preferred trajectories.

Fields with distinct geometries

When two (or more) fields have distinct input geometries (defined here as differing in at least one of the following axes: isocenter, patient support surface angle, or field opening (typically defined by the jaw positions)), the collimator of each field can be optimized independently of each other.

Some scenarios supported by this mode include the following.

When the patient support angle or isocenter axes are different for the different fields, the fields are already delivering dose from distinct directions, so whether the resulting collimator angles are different is usually going to be irrelevant. Furthermore, because of the above, their BEVs are going to be different, so most likely the resulting collimator trajectories will nevertheless be different.

When the user defines different jaw positions for the fields, but the rest of the geometry is identical, the intent of the user is likely to have a split field set up, where each of the arcs is delivering dose to a different part of the target. In this case, by having no penalties, and identical input geometries, the resulting trajectories can be identical, fulfilling the user intent.

User-defined workflow

Finally, if it is so desired, instead of automatically detecting whether the geometries are identical as defined in the previous sections, these teachings will support a user input that can indicate whether to proceed as described above for the distinct collimator per arc case or for the case with no penalties, possibly resulting in identical collimator trajectories.

Further aspects of the invention are provided by the subject matter of the following clauses (where it will be understood that any of these clauses can be combined with any one of more of the other clauses as appropriate).

Clause 1. A method comprising: by a control circuit: optimizing a radiation treatment plan for a particular patient using a particular radiation treatment platform, which radiation treatment plan has a plurality of treatment fields where a source of radiation is moving according to a treatment arc, wherein in at least one of the treatment fields the source of radiation stops at at least one stopping point to deliver dose or additional dose, and wherein optimizing the radiation treatment plan includes optimizing collimator rotation with respect to the at least one stopping point; outputting an optimized radiation treatment plan.

Clause 2. The method of clause 1 wherein optimizing the radiation treatment plan comprises optimizing collimator rotations before initiating dose optimization iterations.

Clause 3. The method of either of clause 1 or 2 wherein the collimator comprises a multi-leaf collimator.

Clause 4. The method of clause 3 wherein optimizing collimator rotation with respect to the at least one stopping point comprises maximizing or determining optimal leaf positioning for the multi-leaf collimator.

Clause 5. The method of any of clauses 1 through 4 wherein optimizing collimator rotation with respect to the at least one stopping point comprises optimizing collimator rotation without physical constraints.

Clause 6. The method of clause 5 wherein optimizing collimator rotation without physical constraints further comprises optimizing by applying weighting that emphasizes the collimator angle at the at least one stopping point.

Clause 7. The method of any of clauses 1 through 6 wherein optimizing collimator rotation with respect to the at least one stopping point comprises optimizing collimator rotation subject to physical constraints regarding at least one of: a collimator rotation value at a static angle; a collimator rotation value at an arc stop position; a collimator rotation value at an arc start position.

Clause 8. The method of any of clauses 1 through 7 wherein optimizing collimator rotation with respect to the at least one stopping point comprises optimizing collimator rotation such that a patient target volume width in a direction coincident with collimator leaves, such as in a direction coincident with a direction of movement of collimator leaves, is minimized.

Clause 9. The method of any of clauses 1 through 8 wherein optimizing collimator rotation with respect to the at least one stopping point comprises: accessing a two-dimensional matrix comprising a cost map having a first axis that corresponds to gantry angles and a second axis that corresponds to collimator angles, such that cells in the two-dimensional matrix store a cost that corresponds to a given combination of gantry angle and collimator angle, (optionally such that collimator angle constraints for static gantry angles and arc start and stop angles are modeled as forbidden or high-cost cells); and determining an optimal path through the two-dimensional matrix, for example such as for at least the most applicable gantry angles.

Clause 10. The method of any of clauses 1 through 9 wherein optimizing collimator rotation with respect to the at least one stopping point comprises optimizing the collimator rotation to favor orienting collimator leaves to yield an emphasized effective radiation modulation of a target volume.

Clause 11. The method of any of clauses 1 through 10 further comprising: administering therapeutic radiation to the particular patient using the optimized radiation treatment plan.

Clause 12. An apparatus comprising: a control circuit configured to: optimize a radiation treatment plan for a particular patient using a particular radiation treatment platform, which radiation treatment plan has treatment fields where a source of radiation is moving according to a treatment arc, wherein in at least one of the treatment fields the source of radiation stops at at least one stopping point to deliver dose or additional dose, and wherein optimizing the radiation treatment plan includes optimizing collimator rotation with respect to the at least one stopping point; output an optimized radiation treatment plan.

Clause 13. The apparatus of clause 12 wherein the control circuit is configured to optimize the radiation treatment plan by optimizing collimator rotations before initiating dose optimization iterations.

Clause 14. The apparatus of either of clause 12 or 13 wherein the collimator comprises a multi-leaf collimator.

Clause 15. The apparatus of clause 14 wherein the control circuit is configured to optimize collimator rotation with respect to the at least one stopping point by maximizing or determining optimal leaf positioning for the multi-leaf collimator.

Clause 16. The apparatus of any of clauses 12 through 15 wherein the control circuit is configured to optimize collimator rotation with respect to the at least one stopping point by optimizing collimator rotation without physical constraints.

Clause 17. The apparatus of clause 16 wherein the control circuit is configured to optimize collimator rotation without physical constraints by applying weighting that emphasizes the collimator angle for the at least one stopping point.

Clause 18. The apparatus of any of clauses 12 through 17 wherein the control circuit is configured to optimize collimator rotation with respect to the at least one stopping point by optimizing collimator rotation subject to physical constraints regarding at least one of: a collimator rotation value at a static angle; a collimator rotation value at an arc stop position; a collimator rotation value at an arc start position.

Clause 19. The apparatus of any of clauses 12 through 18 wherein the control circuit is configured to optimize collimator rotation with respect to the at least one stopping point by optimizing collimator rotation such that a patient target volume width in a direction coincident with collimator leaves, and in an optional example in a direction coincident with a direction of movement of collimator leaves, is minimized.

Clause 20. The apparatus of any of clauses 12 through 19 wherein the control circuit is configured to optimize collimator rotation with respect to the at least one stopping point by: accessing a two-dimensional matrix comprising a cost map having a first axis that corresponds to gantry angles and a second axis that corresponds to collimator angles, such that cells in the two-dimensional matrix store a cost that corresponds to a given combination of gantry angle and collimator angle, (optionally such that collimator angle constraints for static gantry angles and arc start and stop angles are modeled as forbidden or high-cost cells); and determining an optimal path through the two-dimensional matrix for at least most applicable gantry angles.

Clause 21. The apparatus of any of clauses 12 through 20 wherein the control circuit is configured to optimize collimator rotation with respect to the at least one stopping point by optimizing the collimator rotation to favor orienting collimator leaves to yield an emphasized effective radiation modulation of a target volume.

Clause 22. The apparatus of any of clauses 12 through 21 further configured to cause a radiation treatment system to administer therapeutic radiation to the particular patient using the optimized radiation treatment plan.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

**Claims**

1.    A method comprising:
by a control circuit:

optimizing a radiation treatment plan for a particular patient using a particular radiation treatment platform, which radiation treatment plan has a plurality of treatment fields where a source of radiation is moving according to a treatment arc, wherein in at least one of the treatment fields the source of radiation stops at at least one stopping point to deliver additional dose, and wherein optimizing the radiation treatment plan includes optimizing collimator rotation with respect to the at least one stopping point; and
outputting an optimized radiation treatment plan.

2.    The method of claim 1 wherein optimizing the radiation treatment plan comprises optimizing collimator rotations before initiating dose optimization iterations.

3.    The method of claim 1 or claim 2 wherein the collimator comprises a multi-leaf collimator;
and optionally:
wherein optimizing collimator rotation with respect to the at least one stopping point comprises maximizing optimal leaf positioning for the multi-leaf collimator.

4.    The method of any preceding claim wherein optimizing collimator rotation with respect to the at least one stopping point comprises optimizing collimator rotation without physical constraints;
and optionally:
wherein optimizing collimator rotation without physical constraints further comprises optimizing by applying weighting that emphasizes the collimator angle at the at least one stopping point.

5.    The method of any preceding claim wherein optimizing collimator rotation with respect to the at least one stopping point comprises optimizing collimator rotation subject to physical constraints regarding at least one of:

(i) a collimator rotation value at a static angle;
(ii) a collimator rotation value at an arc stop position; and
(iii) a collimator rotation value at an arc start position.

6.    The method of any preceding claim wherein optimizing collimator rotation with respect to the at least one stopping point comprises optimizing collimator rotation such that a patient target volume width in a direction coincident with collimator leaves is minimized.

7.    The method of any preceding claim wherein optimizing collimator rotation with respect to the at least one stopping point comprises:

accessing a two-dimensional matrix comprising a cost map having a first axis that corresponds to gantry angles and a second axis that corresponds to collimator angles, such that cells in the two-dimensional matrix store a cost that corresponds to a given combination of gantry angle and collimator angle, such that collimator angle constraints for static gantry angles and arc start and stop angles are modeled as forbidden or high-cost cells;
determining an optimal path through the two-dimensional matrix for at least most applicable gantry angles.

8.    The method of any preceding claim wherein optimizing collimator rotation with respect to the at least one stopping point comprises optimizing the collimator rotation to favor orienting collimator leaves to yield an emphasized effective radiation modulation of a target volume.

9.    An apparatus comprising:
a control circuit configured to:

optimize a radiation treatment plan for a particular patient using a particular radiation treatment platform, which radiation treatment plan has treatment fields where a source of radiation is moving according to a treatment arc, wherein in at least one of the treatment fields the source of radiation stops at at least one stopping point to deliver additional dose, and wherein optimizing the radiation treatment plan includes optimizing collimator rotation with respect to the at least one stopping point; and

output an optimized radiation treatment plan.

10. The apparatus of claim 9 wherein the control circuit is configured to optimize the radiation treatment plan by optimizing collimator rotations before initiating dose optimization iterations.

11. The apparatus of claim 9 or claim 10 wherein the collimator comprises a multi-leaf collimator;

and optionally:

wherein the control circuit is configured to optimize collimator rotation with respect to the at least one stopping point by maximizing optimal leaf positioning for the multi-leaf collimator.

12. The apparatus of any of claim 9 to claim 11, wherein the control circuit is configured to optimize collimator rotation with respect to the at least one stopping point by optimizing collimator rotation without physical constraints;

and optionally:

wherein the control circuit is configured to optimize collimator rotation without physical constraints by applying weighting that emphasizes the collimator angle for the at least one stopping point.

13. The apparatus of any of claim 9 to claim 12, wherein the control circuit is configured to optimize collimator rotation with respect to the at least one stopping point by optimizing collimator rotation subject to physical constraints regarding at least one of:

(i) a collimator rotation value at a static angle;
(ii) a collimator rotation value at an arc stop position; and
(iii) a collimator rotation value at an arc start position;

and optionally:

wherein the control circuit is configured to optimize collimator rotation with respect to the at least one stopping point by optimizing collimator rotation such that a patient target volume width in a direction coincident with collimator leaves is minimized.

14. The apparatus of any of claim 9 to claim 13, wherein the control circuit is configured to optimize collimator rotation with respect to the at least one stopping point by:

accessing a two-dimensional matrix comprising a cost map having a first axis that corresponds to gantry angles and a second axis that corresponds to collimator angles, such that cells in the two-dimensional matrix store a cost that corresponds to a given combination of gantry angle and collimator angle, such that collimator angle constraints for static gantry angles and arc start and stop angles are modeled as forbidden or high-cost cells;

determining an optimal path through the two-dimensional matrix for at least most applicable gantry angles.

15. The apparatus of any of claim 9 to claim 14, wherein the control circuit is configured to optimize collimator rotation with respect to the at least one stopping point by optimizing the collimator rotation to favor orienting collimator leaves to yield an emphasized effective radiation modulation of a target volume.

FIG. 1

200

FOR A PARTICULAR PATIENT USING A PARTICULAR RADIATION TREATMENT PLATFORM,WHICH RADIATION TREATMENT PLAN HAS A PLURALITY OF TREATMENT FIELDS WHERE A SOURCE OF RADIATION IS MOVING ACCORDING TO A TREATMENT ARC,WHEREIN IN AT LEAST ONE OF THE TREATMENT FIELDS THE SOURCE OF RADIATION STOPS AT AT LEAST ONE STOPPING POINT TO DELIVER ADDITIONAL DOSE ~201

↓

BY A CONTROL CIRCUIT

↓

OPTIMIZING A RADIATION TREATMENT PLAN WHEREIN OPTIMIZING THE RADIATION TREATMENT PLAN INCLUDES OPTIMIZING COLLIMATOR ROTATION WITH RESPECT TO THE AT LEAST ONE STOPPING POINT ~202

↓

OUTPUTTING AN OPTIMIZED RADIATION TREATMENT PLAN ~203

↓

ADMINISTERING THERAPEUTIC RADIATION TO THE PARTICULAR PATIENT USING THE OPTIMIZED RADIATION TREATMENT PLAN ~204

**FIG. 2**

300

OPTIMIZING COLLIMATOR ROTATION WITH RESPECT TO THE AT LEAST ONE STOPPING POINT

↓

ACCESSING A TWO-DIMENSIONAL MATRIX COMPRISING A COST MAP HAVING A FIRST AXIS THAT CORRESPONDS TO GANTRY ANGLES AND A SECOND AXIS THAT CORRESPONDS TO COLLIMATOR ANGLES,SUCH THAT CELLS IN THE TWO-DIMENSIONAL MATRIX STORE A COST THAT CORRESPONDS TO A GIVEN COMBINATION OF GANTRY ANGLE AND COLLIMATOR ANGLE,SUCH THAT COLLIMATOR ANGLE CONSTRAINTS FOR STATIC GANTRY ANGLES AND ARC START AND STOP ANGLES ARE MODELED AS FORBIDDEN OR HIGH-COST CELLS ~301

↓

DETERMINING AN OPTIMAL PATH THROUGH THE TWO-DIMENSIONAL MATRIX FOR AT LEAST MOST APPLICABLE GANTRY ANGLES ~302

**FIG. 3**

Europäisches
Patentamt

European
Patent Office

Office européen
des brevets

# EUROPEAN SEARCH REPORT

**Application Number**

EP 25 20 4593

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/101322 A1 (OLLILA SANTTU [FI] ET AL) 2 April 2020 (2020-04-02) * paragraphs [0054], [0055], [0158] - [0187], [0239], [0261] * * paragraphs [0290] - [0304]; claims 2,3; figures 18-23 * | 1-15 | INV. A61N5/10 |
| Y | US 2020/054895 A1 (RANGANATHAN VAITHEESWARAN [IN]) 20 February 2020 (2020-02-20) * paragraphs [0002] - [0043]; claims 1-20; figures 1-5 * | 1-15 | |
| Y | EP 4 272 814 A1 (RAYSEARCH LAB AB [SE]) 8 November 2023 (2023-11-08) * paragraphs [0026] - [0028], [0076]; claims 1-18 * | 1-15 | |
| Y | US 2019/209864 A1 (STAHL JOHANNES [US] ET AL) 11 July 2019 (2019-07-11) * paragraphs [0013], [0046], [0059], [0065], [0073] - [0090] * | 1-15 | TECHNICAL FIELDS SEARCHED (IPC) A61N |
| Y | US 2019/030372 A1 (MACDONALD ROBERT LEE [CA] ET AL) 31 January 2019 (2019-01-31) * paragraphs [0095], [0127] - [0128] * | 1-15 | |
| Y | US 2023/347174 A1 (HAN MENGRONG [CN] ET AL) 2 November 2023 (2023-11-02) * paragraphs [0006], [0056], [0057], [0095] - [0104], [0159] - [0161]; claim 3 * | 1-15 | |
| X,P | EP 4 563 197 A1 (SIEMENS HEALTHINEERS INT AG [CH]) 4 June 2025 (2025-06-04) * lines 27,29,38,55-75; figures 1-5 * | 1,9 | |

The present search report has been drawn up for all claims

| Place of search | Date of completion of the search | Examiner |
|---|---|---|
| Munich | 9 February 2026 | Rodríguez Cosío, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 20 4593

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2026

| Patent document cited in search report | | Publication date | Patent family member(s) | | Publication date |
|---|---|---|---|---|---|
| US 2020101322 | A1 | 02-04-2020 | CN | 112805059 A | 14-05-2021 |
| | | | EP | 3856336 A2 | 04-08-2021 |
| | | | US | 2020101322 A1 | 02-04-2020 |
| | | | US | 2020101323 A1 | 02-04-2020 |
| | | | US | 2020101325 A1 | 02-04-2020 |
| | | | US | 2022339468 A1 | 27-10-2022 |
| | | | US | 2022370829 A1 | 24-11-2022 |
| | | | US | 2023338748 A1 | 26-10-2023 |
| | | | US | 2024001146 A1 | 04-01-2024 |
| | | | WO | 2020069421 A2 | 02-04-2020 |
| US 2020054895 | A1 | 20-02-2020 | CN | 109923615 A | 21-06-2019 |
| | | | EP | 3535760 A1 | 11-09-2019 |
| | | | US | 2020054895 A1 | 20-02-2020 |
| | | | WO | 2018083072 A1 | 11-05-2018 |
| EP 4272814 | A1 | 08-11-2023 | CN | 119095649 A | 06-12-2024 |
| | | | EP | 4272814 A1 | 08-11-2023 |
| | | | JP | 2025514925 A | 13-05-2025 |
| | | | US | 2025108232 A1 | 03-04-2025 |
| | | | WO | 2023213877 A1 | 09-11-2023 |
| US 2019209864 | A1 | 11-07-2019 | CN | 109496160 A | 19-03-2019 |
| | | | US | 2019209864 A1 | 11-07-2019 |
| | | | US | 2021252311 A1 | 19-08-2021 |
| | | | US | 2023105143 A1 | 06-04-2023 |
| | | | US | 2024207646 A1 | 27-06-2024 |
| | | | WO | 2019134153 A1 | 11-07-2019 |
| US 2019030372 | A1 | 31-01-2019 | EP | 3426344 A1 | 16-01-2019 |
| | | | US | 2019030372 A1 | 31-01-2019 |
| | | | WO | 2017152286 A1 | 14-09-2017 |
| US 2023347174 | A1 | 02-11-2023 | NONE | | |
| EP 4563197 | A1 | 04-06-2025 | CN | 120053903 A | 30-05-2025 |
| | | | EP | 4563197 A1 | 04-06-2025 |
| | | | JP | 2025102625 A | 08-07-2025 |
| | | | US | 2025170420 A1 | 29-05-2025 |

EPO FORM P0459

For more details about this annex : see Official Journal of the European Patent Office, No. 12/82